# EUROPEAN PATENT SPECIFICATION

(11) **EP 2 603 247 B1**
(45) Date of publication and mention of the grant of the patent: **19.11.2014**
(21) Application number: 11764341.1
(22) Date of filing: 10.08.2011
(51) Int. Cl.: A61L 17/00, A61L 27/22, A61K 9/00

(54) **FIBRIN BASED SCAFFOLD, PREPARATION AND USE THEREOF**
FIBRINBASIERTES GERÜST, HERSTELLUNG UND VERWENDUNG
ECHAFAUDAGE À BASE DE FIBRINE, SA PRÉPARATION ET SON UTILISATION

(30) Priority: 12.08.2010 IL 20758610; 12.08.2010 US 372929 P
(43) Date of publication of application: 19.06.2013
(73) Proprietor: Omrix Biopharmaceuticals Ltd., Rehovot 76106 (IL)
(72) Inventor: ATLAS, Roee, Givatayim 53101 (IL); NUR, Israel, 79860 Moshav Timmorim (IL); MEIDLER. Roberto, Rehovot 76447 (IL); BAR, Liliana, Rehovot 76247 (IL); BUENSUCESO, Charito, North Brunswick, NJ 08902 (US); KIHM, Anthony J., Princeton, New Jersey 08540 (US); DHANARAJ, Sridevi, Raritan, New Jersey 08869 (US)
(74) Representative: Kirsch, Susan Edith
(86) International application number: PCT/IL2011/000653
(87) International publication number: WO 2012/020412

(56) References cited:
- EP-A2- 0 339 607
- WO-A1-2009/017267
- WO-A2-2010/006219
- US-A1- 2009 214 649
- DATABASE WPI Thomson Scientific, London, GB; AN 2007-374928 XP002670178, CHEN S; HU J; HUANG A; JIN D; PEI G; ZENG X; ZANG Y: "INJECTION TISSUE ENGINEERING BONE CONSTRUCTION APPLY", -& CN 1 846 790 A (NANFANG HOSPITAL) 18 October 2006 (2006-10-18)
- CATELAS ET AL.: TISSUE ENG, vol. 12, 2006, pages 2385-2396, XP055019982,

## Description

### Field of the invention

The invention relates to a fibrin based scaffold suitable for cell therapy.

### Background of the invention

In recent years, numerous therapies have been developed utilizing a variety of stem cells, which are at the heart of an emerging new specialty called regenerative medicine. Regenerative medicine has the potential to harness stem cells from embryonic and adult sources to provide replacement cell therapies in genetic, malignant, and degenerative conditions.

Studies regarding regenerative medicine have been developed in two main directions: studies directed towards regeneration of cells or tissues by using exogenous administration of biological or chemical factors such as growth factors to induce endogenous regeneration of a damaged tissue or whole organ (for example use of VEGF administration for induction of new blood vessels); and studies on replenishment of damaged tissue or whole organs by exogenous cells and stem cells transplantation (such as bone marrow transplantation), including cells seeded on various scaffold that enhance engraftment of the cells *in vivo,* wound healing, and repair of the skin or organs damaged by surgical operations (Hunt NC, Grover LM. Biotechnol Lett. 2010 Jun; 32(6):733-42; Spotnitz WD. World J Surg. 2010 Apr;34(4):632-4). In regenerative medicine "cells" and "scaffold" are used in combination for regeneration of a tissue. Typically, scaffold materials have two characteristics; they are biocompatible, whereby the materials do not harm a living body, and are bioabsorbable, whereby the materials are decomposed and absorbed in the living body when new living tissues are formed (Uibo R, Laidmäe I, Sawyer ES, Flanagan LA, Georges PC, Winer JP, Janmey PA. Biochim Biophys Acta. 2009 May;1793(5):924-30). Some biological materials have both characteristics and can be used as scaffold in tissue regeneration (Chajra H, Rousseau CF, Cortial D, Ronzière MC, Herbage D, Mallein-Gerin F, Freyria AM. Biomed Mater Eng. 2008;18(1 Suppl):S33-45).

Direct injection or transplantation of cells may effectively restore small areas of damage, but reconstruction of severe damage of injured tissue, for example damage resulting from vascular blockage, extensive therapy with numerous endothelial cells is required. Cell therapy efficiency can be enhanced by maintaining endothelial cells at a target site for a therapeutically effective period of time. Reported are studies on retention of cells inside muscle settings which indicate a therapeutically effective period of time of 4 days or more (Peirce SM, Skalak TC Microcirculation. 2003 Jan;10(1):99-111).

Timothy P. Martens,et al., Cell Transplant. 2009; 18(3): 297-304; Nakamuta JS et al., PLoS One. 2009 Jun 23;4(6):e6005, Christman KL et al., J Am Coll Cardiol. 2004 Aug 4;44(3):654-60, and others proved that implanting cells directly in muscle is inefficient. These studies showed that the implanted cells are rapidly cleared from the implantation site or die about 24 hours after implantation. Typically, the cells used for implantation are stem cells capable of releasing beneficial growth factors/cytokines. However, in view of the above, injection of cells directly to the implantation site cannot allow the release of growth factors and cytokines into the site of implant for a sustained period of time. These studies also showed that administration of the cells embedded in polymers of various kinds can greatly assist in retaining the cells in the implantation site.

The fast clearance of the cells from the site of implantation and/or death of the cells can be attributed to one or more of the following factors: blood flow or body fluids that wash the cells from the implantation site, scarcity of nutrients such as growth factors, inadequate pH, lack of oxygen in the wound area, innate immune reaction (complement) and phagocyt activity of the immune system (Guo S, Dipietro LA J Dent Res. 2010 Mar;89(3):219-29). The fast clearance of injected cells spurred the development of a suitable scaffold that can anchor the cells to the injection site and support cells viability for the first days so that the cells can exert their trophic effect to the ischemic area, *inter-alia*, by releasing of beneficial levels of growth factors/cytokines for a sustained period of time. As the wounded area heals and new blood vessels growth (angiogenesis) are in process, a process that takes few days to 2 weeks (Guo S, Dipietro LA J Dent Res. 2010 Mar;89(3):219-29), the ability of the cells to release cytokines in a sustained manner locally, e.g. at the site of ischemia, is important for efficient healing (Guo S, Dipietro LA J Dent Res. 2010 Mar;89(3):219-29).

Various biological materials have been used as cell scaffolds for cell implantation. Among these materials are collagen, gelatin, elastin, hyaluronic acid, alginate, chitosan, and fibrin (Hunt NC, Grover LM Biotechnol Lett. 2010 Jun;32(6):733-42).

The scaffold material that supports the cells advantageously meets several important criteria. For example, the material is biocompatible, so as not to be toxic or injurious, and not cause immunological rejection. Also, the material is biodegradable. Importantly, the scaffold has the ability to withstand, for a sufficient period of time, its degradation inside the body environment. For example, a sufficient period of time is a time that allows the cells within the scaffold to exert prolonged and local release of growth factors/cytokines. Fibrinogen is a soluble protein found in the blood plasma of all vertebrates. When fibrinogen and factor XIII are contacted by thrombin, a proteolytic enzyme, the fibrinogen is converted into fibrin monomers and Factor XIII is activated to Factor XIIIa. Factor XIIIa then polymerizes the fibrin monomer to form a stabilized fibrin network polymer. Such a network is essential to the healing process of wound closure and tissue bonding. The network serves as a physical bonding material and as a scaffold to epithelial cells or immunologically active cells that migrate to the wound and contribute to tissue regeneration and repair or to defense against invading pathogens. Eventually, the fibrin network is gradually dissolved by the body (a process oftentimes named fibrinolysis).

Fibrin sealants are used extensively in surgical settings for facilitating hemostasis and to reduce operative and postoperative bleeding when other surgical mechanical methods are inappropriate. They are used for example during liver surgery such as liver resection and knee and hip replacement surgery. At present, commercial fibrin sealant is widely used in various types of surgical operations to stop bleeding. In addition, a large number of techniques employing the use of such fibrin sealant as a biological scaffold in regenerative medicine have been reported (cell Transplantation, 7, 309-317, 1998; and Burn, 24, 621-630,1998).

Typically, fibrin sealants consist of two human plasma-derived components: e.g. (a) a highly concentrated Fibrinogen mixture primarily composed of fibrinogen along with catalytic amounts of Factor XIII and (b) a high potency thrombin. Generally, the clot (or fibrin gel) formed after mixing the two components adheres well to wound and tissue surfaces, which leads to the adhesive and haemostatic effect (U.S. Pat. No. 7,241,603). Fibrin gels have been shown to induce growth and osteogenic differentiation of embedded mesenchymal stem cells (MSC), depending on the concentrations of FC and thrombin present in the matrix (Catelas et al., Tissue Eng 12:2385-2396, 2006).

A more recent publication indicates that the characteristics of fibrin hydrogels can be optimized to support specific type of cells for regeneration of a desired tissue (Tamer et al., Tissue Eng 14: 1-17, 2008).

Therefore, there is an unmet need for a fibrin based scaffold capable of anchoring specific type of cells to an implantation site, e.g. an ischemic or near infarct regions, for a therapeutically effective period of time to facilitate blood vessel formation.

### Summary of the invention

In one aspect, the invention relates to a fibrin based scaffold capable of supporting a population of cells embedded in the scaffold comprising at least 1 x 10⁶ cells/ml scaffold mesenchymal stem cells (MSC) and/or mammalian umbilical tissue derived cells (UTC), wherein the scaffold is formed with a fibrinogen component comprising fibrinogen having a final concentration of higher than 17.5 mg/ml scaffold,
the scaffold comprises embedded UTC; MSC; or a combination thereof, in the range of 1 x 10⁶ to 6 x 10⁶ cells/ml scaffold,
wherein the fibrin based scaffold is substantially free of added protease inhibitors, and wherein the scaffold comprising embedded MSC and/or UTC is for use in increasing vascularisation.

Yet in another embodiment of the invention, the fibrinogen component is blood derived.

In still another embodiment of the invention, the scaffold is substantially free of plasmin and/or plasminogen.

In another aspect, the product is for use in a method for increasing vascularization at a confined region of a subject in need comprising administering to the region or forming in the region a scaffold according to the invention that comprises embedded UTC and/or MSC.

In one embodiment of the invention, the region is ischemic, damaged or comprises a transplanted tissue (e.g. a muscle tissue) or organ.

In another aspect, the product is for use in a method for treating a wound comprising administering or forming proximal to the wound a scaffold according to the invention that comprises embedded UTC and/or MSC.

In one embodiment of the invention, the scaffold is capable of supporting the cells at the region or proximal to the wound for a therapeutically effective period of time (e.g. 4 days or more).

In another aspect, the invention relates to a method or forming a scaffold for use in increasing vascularisation, said method comprising the steps of: providing a fibrinogen component; providing a proteolytic enzyme component which is capable of forming fibrin when it reacts with fibrinogen; providing a population of cells comprising MSC and/or UTC; and mixing the fibrinogen, the proteolytic enzyme, and the cells, wherein the formed scaffold comprises at least 1 x 10⁶ cells/ml scaffold MSC and/or UTC and wherein the fibrinogen component used to form the scaffold is in a final concentration of higher than 17.5 mg/ml scaffold, wherein the concentration of the UTC and/or MSC is in the range of 1 x 10⁶ to 6 x 10⁶ cells/ml scaffold, and
the components are substantially free of added protease inhibitors.

In a further embodiment of the invention, the fibrinogen is blood derived and is substantially free of plasmin and/or plasminogen.

In another aspect, the invention provides a kit for use in increasing vascularisation comprising:
(i) at least two separate containers comprising components required to form a fibrin scaffold, the at least one separated container comprises a fibrinogen component having a final fibrinogen concentration of higher than 17.5 mg/ml scaffold formed, and the at least second separated container comprises a proteolytic enzyme component which is capable of forming fibrin when it reacts with fibrinogen; and (ii) MSC and/or UTC at a concentration of at least 1 x 10⁶ to 6 x 10⁶ cells/ml scaffold formed, wherein the components are substantially free of added protease inhibitors.

The scaffold according to the invention can be used for increasing vascularization at a confined region and/or for treating a wound.

### Brief description of the drawings

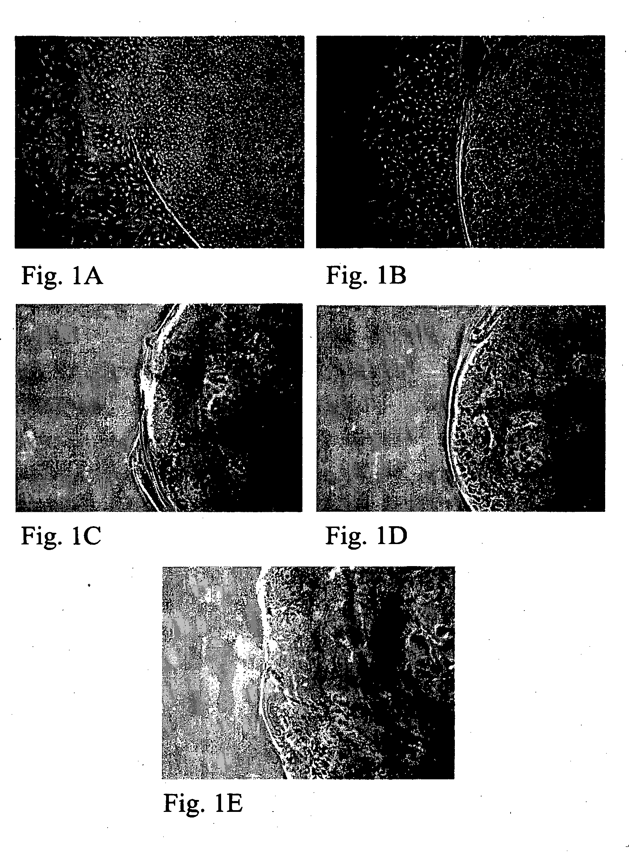

Figs. 1A-1E show representative phase contrast images of HUVEC in the different fibrinogen concentrations used to prepare the fibrin scaffold: 1 (A), 10 (B); 30 (C); 50 (D); or 70 (E) mg/ml (the final fibrinogen concentration following mixing of the two components was half i.e. 0.5, 5, 15, 25 or 35 mg/ml).

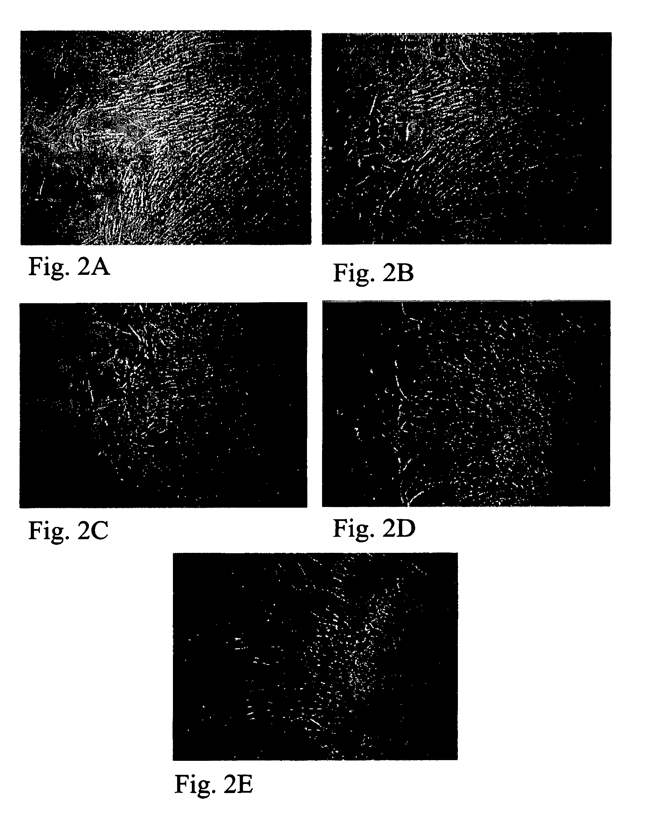

Figs. 2A-2E show representative phase contrast images of human umbilical tissue derived cells (hUTC) in the different fibrinogen concentrations used to prepare the fibrin scaffold: 1 (A), 10 (B); 30 (C); 50 (D); or 70 (E) mg/ml (the final fibrinogen concentration following mixing of the two components was half i.e. 0.5, 5, 15, 25 or 35 mg/ml).

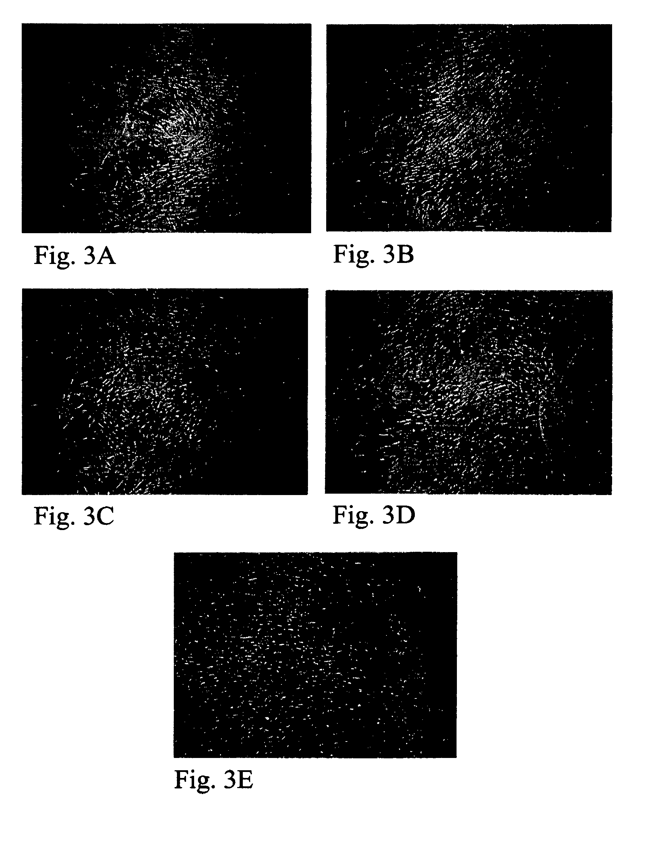

Figs. 3A-3E show representative phase contrast images of MSC in the different fibrinogen concentrations used to prepare the fibrin scaffold: 1 (A), 10 (B); 30 (C); 50 (D); or 70 (E) mg/ml (the final fibrinogen concentration following mixing of the two components was half i.e. 0.5, 5, 15, 25 or 35 mg/ml).

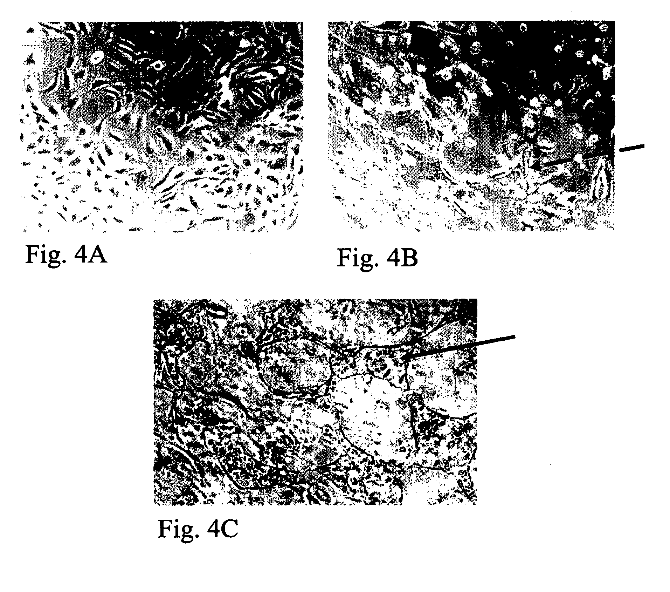

Figs. 4A-4C show representative microscope phase contrast images (obtained at a X20 magnification) of cell processes (striped arrow) and vessel-like structure (continuous arrow) of hUTC grown within a fibrin scaffold formed from 50 mg/ml (25 mg/ml final) fibrinogen and 20 IU/ml (10 IU/ml final) thrombin. The figure was taken after 2 (B) and 6 (C) days of culture within the scaffold. Fig. 4A shows a representative picture of hUTC grown in monolayer.

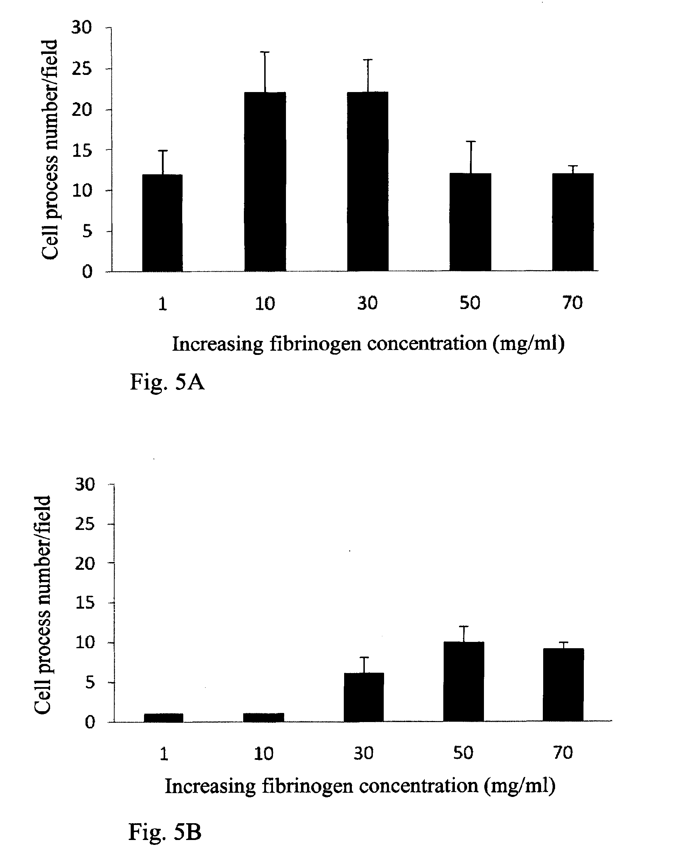

Figs. 5A-5C show the average number of cell process formed per field (1000x1000 microns) when HUVEC (A), hUTC (C), and MSC (B) were cultured in fibrin scaffold formed from increasing fibrinogen concentrations (1-70 mg/ml; 0.5-35 mg/ml final).

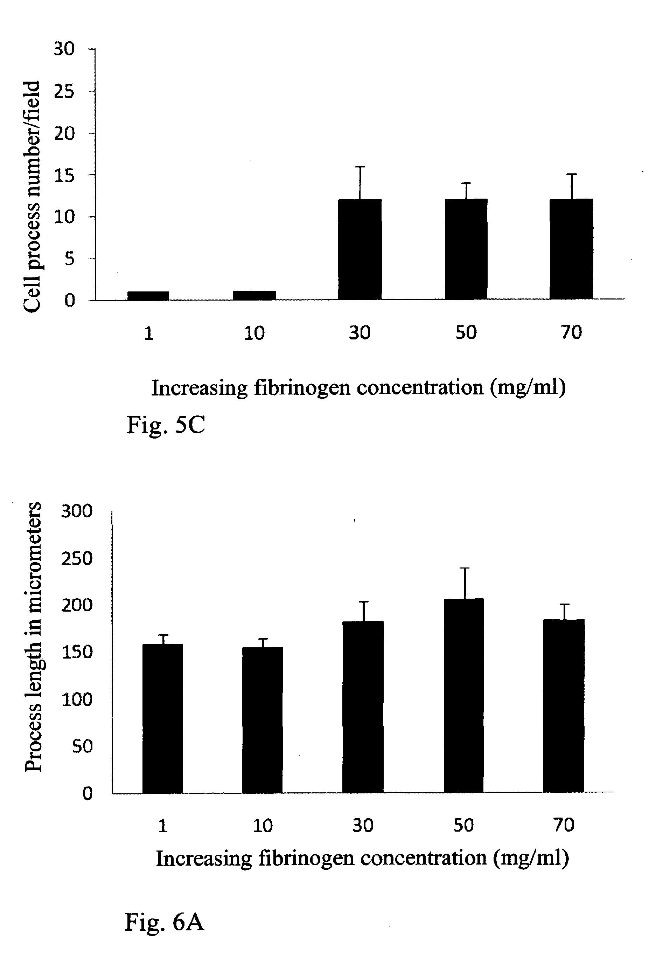

Figs. 6A-6C show the average length of acquired processes in HUVEC (A), hUTC (C), and MSC (B) cultured in fibrin scaffold formed from increasing fibrinogen concentrations (1-70 mg/ml; 0.5-35 mg/ml final).

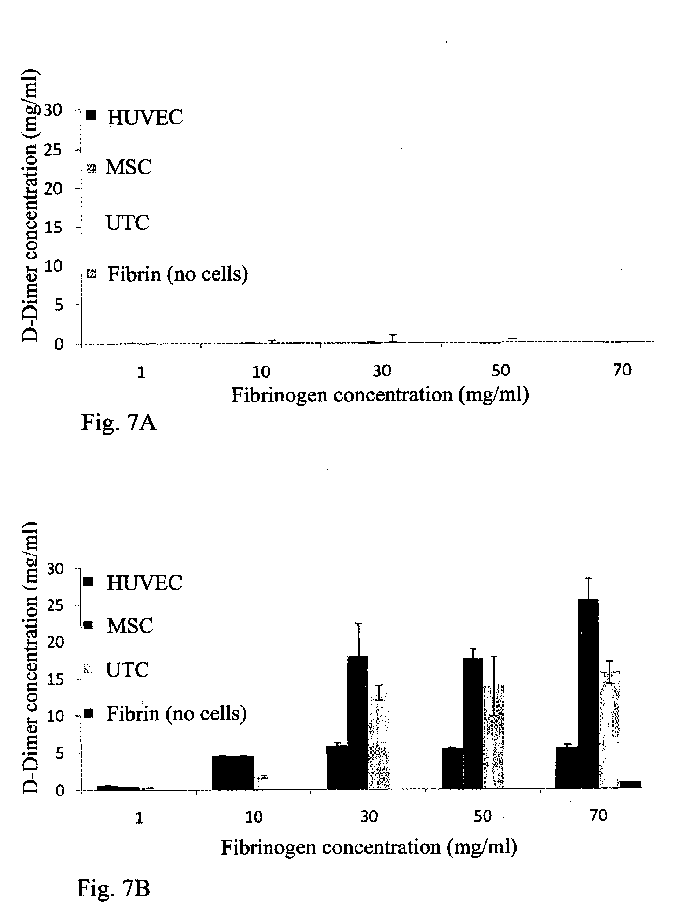

Figs. 7A-7B show the D-dimer concentration level in the medium of HUVEC, MSC and hUTC cultured 1 (A) and 7 (B) days in fibrin scaffold formed with increasing fibrinogen concentrations (and in fibrin scaffold without cells as control).

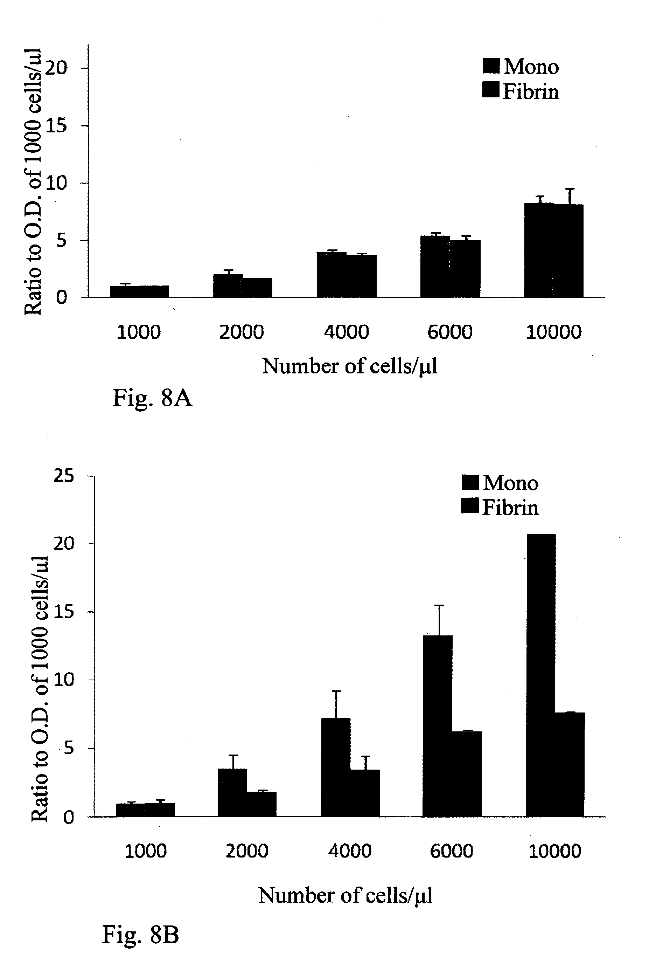

Figs. 8A-8C show increasing cell numbers of HUVEC (A), MSC (B) and hUTC (C) seeded and cultured as a monolayer or embedded in fibrin scaffold.

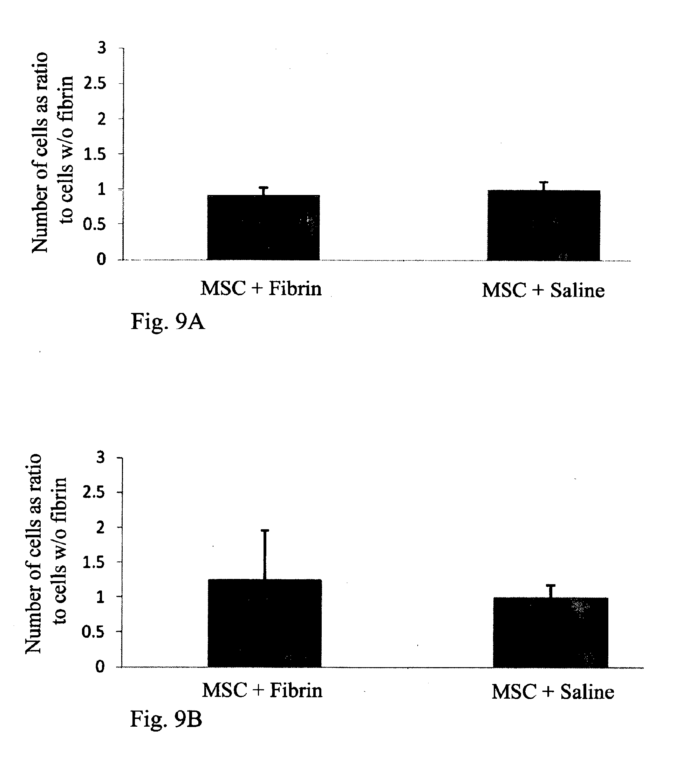

Figs. 9A-9D show the number of MSC present in the injected muscle after 1 day (A, C) and 4 days (B, D) of injection of fibrin + cells {formed with either 17.5 mg/ml fibrinogen final (A) (B)] or 25 mg/ml fibrinogen final (C) (D)} or injection of saline + cells. The counted number of cells in either of the fibrin + cells injected limb or the saline + cells injected limb was divided by the number of cells counted in the saline + cells injected limb on the same day, to obtain a ratio of cells in either limb.

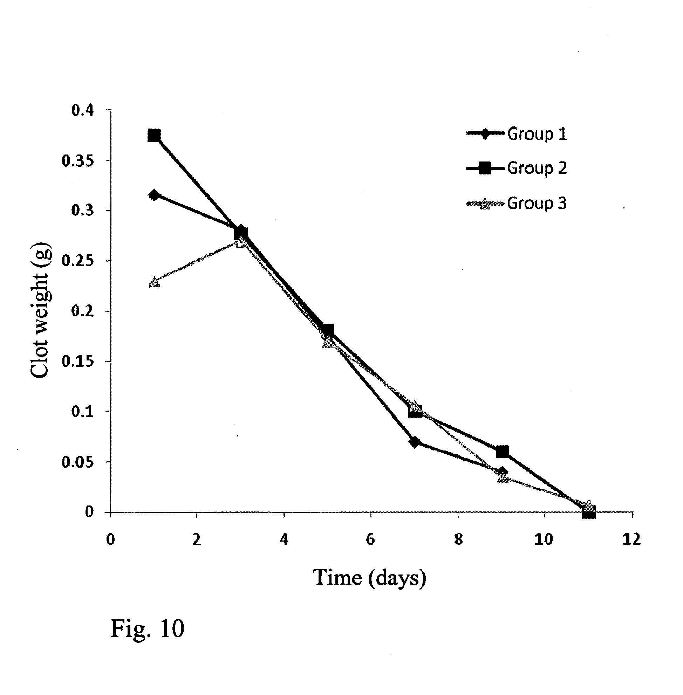

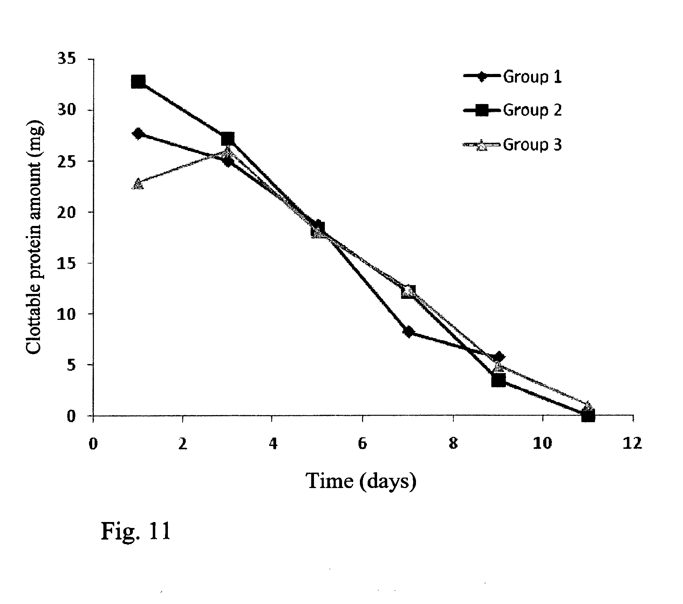

Fig. 10 shows the effect of added protease inhibitors (tranexamic acid or aprotinin) on the longevity of the fibrin clot *in vivo.* Three different fibrin sealant compositions were tested: group 1- without added protease inhibitor; group 2- with tranexamic acid; and group 3- with aprotinin. The three compositions were applied onto a defect created in a rat abdominal muscle wall. The animals in the different groups were sacrificed 1, 3, 5, 7, 9 and 11 days after the initiation of the experiment and the clot's weight was measured. Fig. 11 shows the effect of added protease inhibitors (tranexamic acid or aprotinin) on the longevity of the fibrin clot *in vivo.* Three different fibrin sealant compositions were tested: group 1- without added protease inhibitor; group 2- with tranexamic acid; and group 3- with aprotinin. The three compositions were applied onto a defect created in a rat abdominal muscle wall. The animals in the different groups were sacrificed 1, 3, 5, 7, 9 and 11 days after the initiation of the experiment and the clottable protein amount was measured.

### Detailed description of the invention

In one aspect, the invention relates to a fibrin based scaffold suitable for cell therapy. The fibrin scaffold of the invention is capable of supporting or anchoring embedded UTC and/or MSC to an implantation site for a therapeutically effective period of time. In particular, the fibrin scaffold of the invention comprising the embedded cells is capable to facilitate blood vessel formation at a given implantation site of a subject. The fibrin scaffold of the invention is formed with a fibrinogen component comprising fibrinogen having a final concentration of higher than 17.5 mg/ml scaffold. In particular, the fibrin scaffold of the invention is capable of supporting a population of cells comprising mesenchymal stem cells (MSC) and/or umbilical tissue derived cells (UTC) seeded to a concentration of at least 1 x 10⁶ cells/ml scaffold.

It was surprisingly found according to the present invention that MSC and UTC require *in vitro* a determined concentration of fibrinogen in order to sustain formation of a three-dimensional tissue scaffold and to obtain three-dimensional vessel-like structures. More specifically, it was found according to the invention that MSC and UTC cultured *in vitro* need a final fibrinogen concentration of at least 15 mg/ml scaffold whereas in the case of other cell types e.g. HUVEC or CD34+ stem cells (see WO2010/006219) lower concentrations of fibrinogen can be used such as 0.5 and 12.5 mg/ml scaffold.

Without being bound to the mechanism, it appears that UTC and MSC have a high capability level of fibrin degradation, as compared to HUVEC, probably as a result of a higher fibrinolytic enzymatic activity level in the former cells. Thus, it is beneficial to use fibrin based scaffolds formed with high fibrinogen content and up to about 50 mg/ml (final fibrinogen concentration) when seeding UTC and/or MSC within the scaffolds, to obtain long lasting three-dimensional fibrin scaffolds.

It was also found that the optimal initial cell seeding concentration range in the fibrin scaffold is more stringent in MSC and UTC as compared to other cells (e.g. HUVEC) and is crucial for the ability of the MSC and UTC to grow and proliferate within the fibrin scaffold.

In an *in vivo* setting, it was found that a fibrin scaffold formed with a fibrinogen component at a concentration of higher than 35 mg/ml (17.5 mg/ml final) enabled formation of a scaffold that anchors the cells to the injected muscle tissue for a therapeutically period of time and thus can be advantageously used in tissue engineering. Of note, it was found that addition of protease inhibitors to fibrin scaffolds alone cannot prolong the longevity of the scaffold *in vivo* even in the absence of embedded cells. Thus, when using fibrin scaffolds that comprise cultured UTC and/or MSC in an *in vivo* setting an increased fibrinogen concentration is required than in an *in vitro* setting (higher than 17.5 mg/ml scaffold for *in vivo* as compared to at least 15 mg/ml scaffold for *in vitro* conditions). The term "fibrin based" means that the main component of the scaffold is fibrin but other components such as blood or plasma components (e.g. growth factors, clotting cascade factors, albumin, immunoglobulins, fibronectin, etc.) may be present in the scaffold as well. The term "scaffold" generally refers to a three-dimensional network structure which is capable of supporting or anchoring embedded cells over time, e.g. for a period of about 4 days or more e.g. for 5, 6, 7, 8, 9, 10 days and so on, into the site of implantation.

The terms "capable of supporting cells", "capable of maintaining cells" or "capable of anchoring cells" are used herein to indicate capability to sustain or retain the cells in a specific desired location. Oftentimes, these terms also indicate the capability of facilitating growth, proliferation, and/or differentiation of the cells.

The terms "fibrin scaffold", "fibrin matrix", "fibrin sealant", "fibrin glue", and "fibrin clot" are used herein interchangeably and refer to a three-dimensional network formed from at least a fibrinogen component comprising fibrinogen and a proteolytic enzyme component which is capable of forming fibrin when it reacts with fibrinogen e.g. thrombin and/or a solution obtained from snake venom. In one embodiment of the invention, the proteolytic enzyme is thrombin. The fibrin scaffold of the invention is formed with at least 17.5 mg/ml fibrinogen final concentration such as fibrinogen having a final concentration of 18, 19, 20, 21, 22, 23, 24, 25, 26, 27, 28, 29, 30, 31, 32, 33, 34, 35 and 50 mg/ml scaffold. In one embodiment of the invention, the final fibrinogen concentration is about 25 mg/ml. In another embodiment of the invention, the final fibrinogen concentration is about 35 mg/ml. As used herein, the term "final concentration" refers to the fibrinogen concentration after combining all the components used to form the scaffold.

The fibrinogen and/or the thrombin components can be prepared from an initial blood composition. The blood composition can be whole blood or blood fractions, i.e. a fraction of whole blood such as plasma. The origin of the fibrinogen and thrombin can be autologous whereby they would be manufactured from the patient's own blood, from pooled blood or fractions. It is also possible that part or all of the protein components used to form the scaffold are prepared by recombinant methods.

In one embodiment of the invention, the fibrinogen component is comprised from a biologically active component (BAC) which is a solution of proteins derived from blood plasma which can further comprise anti fibrinolytic agents such as tranexamic acid and/or stabilizers such as arginine, lysine, their pharmaceutically acceptable salts, or mixtures thereof. BAC can be derived from cryoprecipitate, in particular concentrated cryoprecipitate. The term "cryoprecipitate" refers to a blood component which is obtained from frozen plasma prepared from whole blood, recovered plasma or from source plasma which is collected by plasmapheresis. A cryoprecipitate can be obtained when frozen plasma is slowly thawed in the cold, typically at a temperature of 0-4°C, resulting in the formation of precipitate that contains fibrinogen and factor XIII. The precipitate can be collected, for example, by centrifugation and dissolved in a suitable buffer such as a buffer containing 120 mM sodium chloride, 10 mM trisodium citrate, 120 mM glycine, 95 mM arginine hydrochloride, 1 mM calcium chloride. The solution of BAC can comprise additional factors such as for example factor VIII, fibronectin, von Willebrand factor (vWF), vitronectin, etc. for example as described in US 6,121,232 and WO9833533. The composition of BAC can comprise stabilizers such as tranexamic acid and arginine hydrochloride. The amount of tranexamic acid in the solution of BAC can be from about 80 to about 110 mg/ml. The amount of arginine hydrochloride can be from about 15 to about 25 mg/ml.

Optionally, the solution is buffered to a physiological compatible pH value. The buffer can be composed of glycine, sodium citrate, sodium chloride, calcium chloride and water for injection as a vehicle. Glycine can be present in the composition in the amount of from about 6 to about 10 mg/ml, the sodium citrate can be in the range of from about 1 to about 5 mg/ml, sodium chloride can be in the range of from about 5 to about 9 mg/ml and calcium chloride can be in the concentration of about 0.1-0.2 mg/ml.

In one embodiment of the invention, the fibrinogen component is blood derived e.g. BAC composition. In another embodiment of the invention, the concentration of plasminogen and/or plasmin in the blood derived component is lowered and the fibrin scaffold is substantially free of plasmin and/or plasminogen. The removal of plasmin and plasminogen from the blood derived component can be carried out as described in US 7,125,569 and WO02095019.

"A fibrin scaffold substantially free of plasmin and/or plasminogen" refers e.g. to a scaffold comprising a level of plasminogen and/or plasmin that is equal to or lower than 15 µg/mL

The present invention also provides a kit comprising containers comprising (i) at least two separated components required to form a fibrin scaffold, the at least one separated component comprises fibrinogen having a final concentration of higher than 17.5 mg/ml scaffold formed, and the at least second separated component comprises a proteolytic enzyme which is capable of forming fibrin when it reacts with fibrinogen (e.g. thrombin); and (ii) MSC and/or UTC cells at a concentration of at least 1 x 10⁶ cells/ml scaffold formed. The kit can also contain instructions for use to the physician, health care professional, and/or the patient. The containers can be vials or pre-filled syringes. The components can be provided in the kit as a solution or in a solid form, e.g. as lyophilized powder. The lyophilized powder can be reconstituted with carries. The container can be of different sizes and contain different volumes of the components, for example, the components can have a volume of equal to or less than about 5 ml, for example, about 3, 2, 1.5 or 1 ml. The kit may also contain a separate vial of the carrier. The components may be mixed in any desired range of ratios. For example, when the concentration of the fibrinogen in the fibrinogen comprising component is 36-100 mg/ml and the thrombin concentration in the thrombin comprising component is about 800-1200 IU/ml, the two components can be mixed in a ratio of 1:1. In one embodiment, the fibrinogen concentration in the fibrinogen comprising component is in the range of 50-90 mg/ml. The cells can be included in the proteolytic enzyme container, in the fibrinogen container, and/or can be in a separated container. Optionally, a device for application is also included in the kit. The components and the cells of the kit can be kept frozen until use.

The results obtained indicate that addition of protease inhibitor such as tranexamic acid or aprotinin into a fibrin sealant formulation substantially free of plasmin and/or plasminogen used to prepare the scaffold according to the invention in an *in vivo* setting did not promote superior clot longevity as compared to the fibrin sealant formulation without any added protease inhibitor. Accordingly, in one embodiment of the invention, the fibrin scaffold is substantially free of added protease inhibitors or antifibrinolytic agents e.g. tranexamic acid, epsilon amino caproic acid (EACA) or aprotinin. For example, the fibrinogen component used to prepare the scaffold according to the invention has no more than about 15 µg/ml tranexamic acid or epsilon amino caproic acid (EACA), or no more than about 5 KIU/ml aprotinin.

Blood derived components are typically purified from infective particles in order to minimize the potential risk posed by blood-borne pathogens. The purification procedure can be carried out by nanofiltration, solvent/detergent treatment, heat treatment such as, but not limited to, pasteurization, gamma or UVC (< 280 nm) irradiation, by any other method known in the art or combinations thereof. The term "infective particle" refers to a microscopic particle, such as, but not limited to, a micro-organism or a prion, which can infect or propagate in cells of a biological organism. The infective particles can be viral particles. Viral inactivation procedure can be carried out by adding a molecule to the composition or blood fraction prior to and/or during the purification procedure. The added molecules and their products can be removed by gravitation, column chromatography, any other method known in the art or combinations thereof.

The removal of infective particles can be carried out by filtration or by selective absorption methods such as affinity, ion exchange or hydrophobic chromatography. A multi-step viral inactivation procedure can be carried out. For example, the composition can be subjected to solvent/detergent treatment, heat treatment, selective chromatography and nanofiltration.

The term "viral inactivation" refers both to the situation wherein viruses are maintained in the composition but are rendered non-viable (for example, by dissolving their lipid coat), as well as to the physical removal of the viruses from the composition (for example, by size exclusion techniques).

"Solvent detergent (S/D) treatment" typically refers to a process that inactivates envelope-coated viruses by destroying their lipid envelope. The treatment can be carried out by the addition of detergents (such as Triton X-45, Triton X-100 or Tween 80) and solvents [such as tri(n-butyl) phosphate (TnBP), di- or trialkylphosphates]. The solvent-detergent combination used to deactivate lipid coated viruses may be any solvent-detergent combination known in the art such as TnBP and Triton X-100; Tween 80 and Sodium cholate and others. The concentration of the solvents and detergents can be those commonly used in the art, for example, >0.1% TnBP and >0.1% Triton X-100. Typically, the conditions under which the solvent-detergent inactivates the viruses consist of 10-100 mg/ml of solvent-detergent at a pH level ranging from 5-8, and a temperature ranging from 2-37°C for 30 min. to 24 hours. However, other solvent-detergent combinations and suitable conditions will be apparent to any person versed in the art. The bulk of the solvent-detergent used in the S/D treatment can be removed, for example, by using chromatography columns such as hydrophobic interaction chromatography column (HIC) e.g. C-18 silica packing material and SDR (Solvent-Detergent removal) HyperD; protein adsorption matrices such as ion-exchange matrices; affinity matrices; and/or size- exclusion matrices.

"Heat treatment" typically refers to a process by which heat destroys both lipid-enveloped and non-enveloped viruses. "Heat treatment" is interchangeable with the term "pasteurization".

The heat inactivation treatment can be carried out at 60°C for 10 hours. Stabilizers such as sucrose and glycine can be added into the composition during the heat treatment. "Nanofiltration" typically refers to a process by which lipid-enveloped and non-enveloped viruses are excluded from the mixture e.g. by using special nanometer-scale filters such as Planova 20N, 35N and 75N; Viresolve/70^{™}, Viresolve/180^{™}.

The composition can be concentrated by ultra-filtration process. The ultrafiltration can be followed by diafiltration to exchange the buffer. The concentration and dialysis by ultrafiltration and diafiltration, respectively, can be carried out in one step or as two separate steps. The diafiltration can be carried out against any solution which is suitable for human administration.

The term "a population of cells" relates to any cell type originated from any lineage. "A lineage" of a cell defines which cells it is originated from and to which cells it can give rise to. Advantageously, UTC and/or MSC can be co-cultured with other cell types using the scaffold of the invention. The population of cells which can be combined with the fibrin scaffold and the MSC and/or UTC, can be selected based on the intended therapeutic use. For example, in case the scaffold is intended to increase vascularization in critical limb ischemia condition to re-establish blood-flow to an affected area, the selected cell population can be muscle cells and/or cells that can give rise to muscle cells. The term "cells embedded in the scaffold" refers e.g. to cells seeded or encapsulated within the scaffold. For formation of scaffold embedded cells, the cells can be incorporated into one recipient together with the fibrinogen component, into another recipient together with the proteolytic enzyme component or can be in a third recipient as a separated component e.g. in an acceptable carrier which is suitable for the cells and for application to the human or animal body. Then, each of the components can be applied onto a site or region in any order e.g. simultaneously or one after the other and a fibrin scaffold with embedded cells is formed on the site or region when the components are mixed. The scaffold of the invention that comprises embedded cells can be formed *in vivo* at the desired region or *ex vivo* and then placed in the region. *Ex vivo* includes *in vitro* cell culturing in standard growth conditions useful for culturing the cells, for example, a suitable growth medium.

The fibrin based scaffold according to the invention is capable of supporting mesenchymal stem cells (MSC) or umbilical tissue derived cells (UTC) in a concentration of at least 1 x 10⁶ cells per ml scaffold. In one embodiment of the invention, the scaffold comprises embedded UTC and/or MSC cells in an initial concentration of at least 1 x 10⁶ cells per ml scaffold e.g. an initial concentration of 2 x 10⁶, 3 x 10⁶, 4 x 10⁶, 5 x 10⁶ per ml scaffold and so on. In another embodiment of the invention, the scaffold with the embedded UTC and/or MSC further comprises other cell types. "An initial concentration" with regard to the concentration of the embedded UTC and/or MSC relates to the concentration of the seeded cells at the time point when the scaffold is formed. However, the cell concentration can increase over time. It was shown according to the invention that MSC and UTC seeded in a fibrin scaffold according to the invention have a limited capacity to proliferate and grow after a certain cell concentration and that the optimal cell concentration for initial seeding which can still be supported by the fibrin scaffold is up to 6 x 10⁶ cells per ml scaffold. Thus, in certain embodiments of the invention, the initial concentration of the embedded UTC and/or MSC is in the range of 1 x 10⁶ to 6 x 10⁶ cells per ml scaffold. In one embodiment of the invention, the scaffold comprises UTC and MSC in the range of 1 x 10⁶ to 6 x 10⁶ cells per ml scaffold. As used herein the term "mesenchymal stem cells (MSC)" refers to cells that can give rise to a large number of tissue types such as bone, cartilage, adipose tissue, connective tissue, and blood and lymphatic vessels e.g. cells that can give rise to new blood vessels. The MSC can be fetal stem cells found in the organs of fetuses or adult stem cells found in adult or newborn individuals. The MSC can be derived from bone marrow (bone marrow-derived mesenchymal stem cells) and/or from the umbilical cord veins (umbilical cord-derived mesenchymal stem cells). The MSC can be of any mammalian origin e.g. human, rat, primate, porcine and the like.

The term "umbilical tissue derived cells (UTC)" refers, for example, to cells as described in US07510873, US07413734, US07524489, and US07560276. The UTC can be of any mammalian origin e.g. human, rat, primate, porcine and the like. In one embodiment of the invention, the UTC are derived from human umbilicus.

The cells can be cultured in a growth medium *in vitro* and/or otherwise manipulated prior to embedding the cells within the scaffold. Culturing cells *in vitro* and counting cells are known in the art (see e.g. Current Protocols in Molecular Biology Chapter 28 Mammalian Cell Culture). The cells used according to the invention can be autologous, allogenic and/or xenogenic cells. The term "autologous cells" refer to cells originated from the same individual to which they will be re-implanted. The term "allogenic cells" refer to cells originated from the body of a donor of the same species. "Xenogenic cells" are cells isolated from individuals of another species.

In one embodiment of the invention, the MSC and/or UTC that can be supported by the scaffold of the invention do not express the CD34+ marker i.e. they are CD34- cells. "CD34+ cells" are immature cells that express at their surface the CD34 marker.

The results obtained according to the invention show that injection of MSC or UTC with a thrombin component and a fibrinogen component at a final concentration of 25 mg/ml in an *in vivo* model system for assessing cell retention after injection of the cells into a limb muscle resulted in a significant increase in the retention of cells in the injected region, e.g. a limb muscle, 4 days following implantation of the cells as compared to cells injected with saline. These results indicate that the scaffold according to the invention can be beneficially used to anchor the cells to an injected region and prevent their clearance from the site of implantation, and therefore can be advantageously used in tissue engineering.

The term "tissue engineering" typically refers to the use of a combination of suitable biochemical and physio-chemical factors and/or cells to restore, replace, maintain, and/or enhance tissue function or a whole organ. The term "tissue engineering" sometimes is synonymous with the term "cell therapy".

A scaffold according to the invention that comprises embedded UTC and/or MSC can be used in tissue engineering to facilitate local development of blood vessels tissue, e.g. for restoring tissue lost due to trauma or disease, for treating wounds, for speeding healing, for restoring, replacing, maintaining, and/or enhancing tissue function or a whole organ. Thus, in another aspect, the invention provides a method for increasing vascularization at a confined region of a subject in need comprising administering to the region or forming in the region a scaffold according to the invention that comprises embedded UTC and/or MSC.

The term "increasing vascularization" refers to enhancement of blood vessel formation beyond that which would occur naturally in a specific period of time, for example 4 days. The term "vascularization" includes the term "angiogenesis" and "neovascularization". Neovascularization refers to the formation of functional microvascular network. Typically, angiogenesis is mainly characterized by the protrusion and outgrowth of capillary buds and sprouts from pre-existing blood vessels.

The term "confined region" relates to a localized site e.g. the region in which the scaffold was formed in or administered to in the body. Advantageously, the fibrin scaffold according to the invention retains the cells at the transplanted site and supports the cells *in situ* for a therapeutically effective period of time. "A therapeutically effective period of time" may be about 4 days or longer. The term "region" and "site" refer to any area of the body including, but not limited to, a body tissue or an organ where vascularization is insufficient and enhanced blood vessels formation is needed. The region can be an area where blood vessel structure exists such as bone, muscle, skin or heart tissue e.g. an injured vascular area. The region can also be an avascular area such as the meniscus of the knee, spinal cord, intervertebral disc, or any cartilage tissue. The region can also be an area comprising a transplanted tissue or organ where vascularization is required for keeping the transplant functional. In cases of tissue or organ transplant, the scaffold can be formed or administered into the region before, after or together with the transplant. The region can also be an organ that its function may be improved by increased vascularization such as the kidneys. The region can be a damaged or an ischemic tissue. In one embodiment of the invention, the region is a muscle tissue.

The term "ischemic tissue" refers to an inadequate blood supply to an organ or tissues of the body e.g. because of reduced blood flow to the tissue area e.g. due to blockage of the blood vessels. The ischemic region can be located in any organ or tissue of the body such as cardiac tissue, neurologic tissue, muscle tissue or any other tissue suffering from inadequate blood circulation.

The region can be a damaged region. Damage to the region can be caused by fractures, injury, surgery, ulcers, bums, necrosis, inflammation, surgical removal of tissue or otherwise missing of tissue, fibrosis, infection, hypoxia, ischemia, ischemia/reperfusion and any other insult or stress to an organ or a tissue of the body. A region or damaged region refer to an area that would benefit from increased blood vessels formation.

Still in another aspect, the invention relates to a method for treating a wound comprising administering or forming on the wound or proximal to the wound a scaffold according to the invention that comprises a population of embedded cells e.g. UTC and/or MSC.

The term "wound" refers to any tissue or location in need of repair and/or healing including, but not limited to, wounds resulting from diseases, injuries or surgeries. By "proximal" it is meant a localized area which is close enough to the wound wherein application of the scaffold with embedded UTC and/or MSC according to the invention will result in enhanced healing as compared to the naturally healing pattern.

In one embodiment of the invention, the cells are localized at the region of the wound or proximal to the wound for a therapeutically effective period of time. Advantageously, the generation or development of new blood vessels at the region or proximal to the wound will result in increased blood circulation beyond that which would occur naturally. The term "therapeutically effective period of time" refers to a period of time which may be sufficient to allow blood vessel formation in the implanted site. In one embodiment of the invention, the time is about 4 days or more.

Blood vessel formation can be identified, for example, by monitoring morphological changes of the implanted cells such as vessel-like cell structure formation as described in Skovseth et. al., Methods in Molecular Biology 2007; 360:253-265. When certain types of cells such as endothelial cells or endothelial stem cells are grown inside a three-dimensional scaffold such as fibrin polymer scaffold and under certain conditions, the cell body acquires lamellipoedia and fillopedia-like extensions called herein "cell processes". Several cell processes, either from the same cell body or from another cell body, form together a vessel-like structure (Kaijzel et al., J. Thrombosis and Haemostasis 2006 4: 1975-1981). The number of the formed cell processes and the length of the acquired vessel like structure can be measured in a defined square region to monitor the increase in vascularization.

"A subject in need" can be a subject having any condition that would benefit from increased blood flow at a region or proximal to a wound. A subject in need can be a subject suffering from hypoxic heart e.g. when vessels to the heart are blocked; gangrene; diabetes; ischemia including critical limb ischemia; inadequate blood flow; coronary artery disease; atherosclerosis; arteriosclerosis; aortic aneurysm; and cerebrovascular disease. In one embodiment of the invention, the subject in need suffers from critical limb ischemia. The scaffold of the invention that comprises embedded UTC and/or MSC may also be used to prevent or reduce the likelihood of an event of myocardiac infarction and infarct expansion. The scaffold of the invention that comprises embedded UTC and/or MSC, with or without other cell types, may be used to treat a wound or increase vascularization by directly injecting the components required to form the scaffold according to the invention and the cells to the desired anatomic site or by implanting a pre-formed scaffold with embedded UTC and/or MSC into the anatomic site. "Anatomic site" is, for example, a pre-selected site in a mammal where vascularization is required and/or any tissue or location in need of repair and/or healing. The term encompasses the terms "region" and "wound".

### Examples

### Materials and methods:

### Cells and Media employed

hMSC- human mesenchymal stem cells, bone marrow from Lonza Cologne AG, Cat No. PT2501.

HUVEC- human umbilical vein endothelial cells from invitrogen company Cat No. C-003-5C.

hUTC- human Umbilical Tissue Derived Cells are described in US07510873, US07413734; US07524489; US07560276 (Ethicon).

Medium for MSC- Medium MSCBM+ MSCGM SingleQuots (Lonza; Cat No. PT-3238+ PT-4105).

Medium for hUTC- DMEM/1g-D-glucose/L-glutamine/Pyruvate +15% FCS Cat. # 31885 Gibco.

Medium for HUVEC- EGM-2-MV medium cc (Lonza; Cat No. CC-4147).

### Preparation of cell culture.

Cells were thawed from liquid nitrogen. A million frozen cells were added with 1 ml medium (according to the cell type as detailed above) and after thawing, plated in tissue culture plate. Following 2 days of growth in an incubator (37°C and 5% CO₂), the cells were used for the experiments below.

### Cell processes counting and processes length measurements for monitoring formation of vessel-like cell structures.

Monitoring morphological vessel-like cell structure formation is a widely used assay to follow induction of angiogenesis by different substances (Skovseth et. al., "The HUVEC/Matrigel assay: an in vivo assay of human angiogenesis suitable for drug validation". Methods Mol Biol. 2007; 360:253-268). When certain type of cells such as endothelial cells or endothelial stem cells (e.g. HUVEC) are grown inside a three-dimensional scaffold (e.g. fibrin polymer scaffold) and under certain conditions, the cell body acquires lamellipoedia and fillopedia-like extensions. These extensions are called "cell processes". Several cell processes, either from the same cell body or from another cell body, form together a vessel-like structure (Kaijzel et al., "Molecular weight fibrinogen variants determine angiogenesis rate in a fibrin matrix in vitro and in vivo". J Thromb Haemost. 2006: 1975-1981). These vessel-like cell structures formed in a tissue-culture well are considered a standard manifest of angiogenesis and are monitored in assays to follow induction of angiogenesis.

The angiogenesis assay includes counting cell processes in the tissue culture in a defined square region of 500 µm x 500 µm 6 days following cell culturing in fibrin scaffold (prepared as elaborated below). The assay also includes measuring the processes length. The measurements were carried out using a computer program ("Cell_A" Olympus 2009). Fig. 4 shows a representative microscope phase contrast images (obtained at a X20 magnification) of cell processes (striped arrow) and vessel-like structure (continuous arrow) of hUTC grown within a fibrin scaffold formed from 50 mg/ml (25 mg/ml final) fibrinogen and 20 IU/ml (10 IU final) thrombin as described below. The figure was taken after 2 (B) and 6 (C) days in culture. Fig. 4A shows a representative figure of cells grown in monolayer. The figure was taken after 6 days in culture. The measurement of the number of cell processes and their length for each well was carried out in duplicates (in two different areas in the same well).

### Quantification of the levels of D-dimer in the medium.

The following assay was used to test the rate of fibrin degradation carried out by cells originated from various lineages. D-dimer is a fibrin degradation product formed when fibrin polymer is broken down by the enzyme plasmin. Fibrin degradation was assessed by measuring the D-dimer level in the medium of various cells grown within a fibrin scaffold. Different fibrinogen concentrations were used to form the fibrin polymer. For monitoring fibrin degradation carried out by cells, different cell types were seeded in a fibrin scaffold (as described in Example 1 below) and the scaffold was maintained in a culture medium (the medium was not changed during the entire experiment period) for up to 7 days. A sample (20 µl) was taken from the medium 1 and 7 days following seeding of the cells and the D-dimer level was measured using Imuclone D-dimer ELISA Kit (Cat No. 602) according to the manufacturer's protocol. D-dimer concentration measured in the medium of fibrin scaffold prepared in the same manner but without cells and fibrin scaffolds with cells on day 1 were used as control.

### Counting of cells within the fibrin scaffold.

The following assay was used to quantify the cells cultured in the scaffold. The cells were embedded in fibrin scaffolds formed inside a 6-well plate as specified in Example 6. Following 1.5 days in culture 3-4 mg (dissolved in 500 µl PBS) trypsin per ml medium was added into each well (each well contained 4 ml medium), and the plate was incubated at 37°C in a humidified atmosphere of 5% CO₂ for 20-30 minutes until the fibrin scaffold was fully degraded. The content of the well was transferred into an eppendorf tube and centrifuged in a microfuge at 1000g for 4 minutes at room temperature (about 22°C). The 4 ml volume of supernatant was discarded, and 1 ml fresh cell medium containing 1 µg/ml Hoechst stain solution (33342 B2261-100 MG Sigma) in 0.4 ml cell growth medium (according to cell type) was added to the cell pellet and the pellet was re-suspended. The above cell suspension in the eppendorf tube was then incubated at 37°C in a humidified atmosphere of 5% CO₂ for 1 hour. Next, the cells were washed once by gentle resuspension of the cells in 1 ml PBS, centrifuged at 1000 g at room temperature and the supernatant was discarded. The cell pellet was resuspended in 200 µl PBS, transferred to a 96-well plate and the absorbance was measured in a spectrophotometer at a wavelength of 350ex/450em nm. The results are presented in OD units relative to the OD of the minimal concentration of cells (1000 cells/µl).

### In vivo cell retention assay.

The following model was used to explore the ability of the fibrin scaffold to retain the transplanted cells within the injected region in an animal model.

A mouse limb muscle cell injection model was used as an *in vivo* model system for studying cell retention after injection of the cells into a limb muscle (Terrovitis J et al., J Am Coll Cardiol. 2009 Oct 20;54(17):1619-26).

Prior to implantation, the cells (HUVEC, MSC, or hUTC) were stained with DiI [a fluorescent molecule that attaches to the cell membrane of viable cells and can be viewed by 570ex/610em nm (Molecular Probes, USA, Cat No. C7001)] as follows: about 330,000 cells were incubated with 2 µM DiI solution in a final volume of 150 µl medium. The staining procedure takes 15 minutes as specified by the manufacturer's manual. After the staining step, the cells were washed once with PBS (by gentle resuspension of the cells in 1ml PBS, centrifugation at 1000 g at room temperature and discarding the supernatant) and the dyed cells were used for the *in vivo* injection procedure as elaborated below.

1-3 months old BalbC mice were injected directly into the limb adductor muscle with 330,000 dyed-cells (suspended in 150 µl saline or in 150 µl fibrin sealant components [i.e. 75 µl thrombin component (20 IU/ml or 10 IU/ml final concentration following mixing of the two components) containing the dyed-cells and 75 µl fibrinogen component (35 or 50 mg/ml; 17.5 or 25 mg/ml final fibrinogen concentration following mixing of the two components). Each mouse was injected in one limb with cells suspended in saline and in the other limb with cells mixed with the fibrin sealant components. Each injection was carried out with a 0.5 ml syringe equipped with a 26G needle. The thrombin component+cells and the fibrinogen component were mixed together in an eppendorf tube using a pipettor, immediately drawn by the syringe and injected into the limb before clotting occured.

The mice were sacrificed 1 or 4 days after injection and the fibrin clot (from the limb injected with cells suspended in the fibrin sealant components) or a piece of muscle (from the limb injected with cells suspended in saline) (0.2x0.2x0.2 cm) was excised from the injection site using a scalpel. The excised fibrin clot pieces or muscle pieces were enzymatically digested to produce a suspension of cells by 3 mg/ml trypsin (only for fibrin clot dissociation) or 6 mg/ml collagenase (for muscle dissociation- added for muscle dissociation in both tested groups) (Sigma, Saint-Louis USA Cat No. T4665 and Cat No. C6079, respectively, dissolved in 400 µl DMEM medium) for 2 hours. at 37°C. Next, the suspension of cells was centrifuged and resuspended in fresh PBS (400 µl). A 400 µl sample of cell suspension from each digested fibrin clot or muscle tissue was transferred to a 24-well plate, 2% paraformaldehyde were added (Sigma, Saint-Louis USA Cat. # 47608) to each well and the cells were counted under a fluorescent microscope at a wavelength of 553ex/570em nm.

### Clot longevity assay in an in vivo setting.

The following assay was used to test the effect of added protease inhibitor on the longevity of the fibrin clot in an *in vivo* setting. The rat abdominal wall defect model was used. Three different fibrin glue compositions were tested: EVICEL (Omrix Biopharmaceuticals Ltd.) without any added protease inhibitor (group number 1), EVICEL with the addition of 100 mg/ml tranexamic acid into the thrombin component (group number 2), and EVICEL with the addition of 3,000 KIU/ml aprotinin into the thrombin component (group number 3). Following the addition of tranexamic acid (Daiichi, Japan) or aprotinin (Sigma-Aldrich, USA Cat No. A1153) the thrombin solution was sterile filtered.

The experiment was carried out in Spargue-Dawley male rats weighing 300-400 g and at the age of over 9 months (Harlan lab. Rehovot, Israel). All animals underwent clinical inspection upon arrival and were continuously under clinical supervision for the experimental period. Allocation to the treatment groups was carried out using a random stratified procedure. The animals were housed in a polycarbonate cage (2 animals per cage) in an air-conditioned room in a temperature of 22±4°C, relative humidity of 30-70% and under an artificial lighting cycle (12 hours artificial light /12 hours dark). The animals had free access to food and sterilized tap water.

Prior to surgery the animals were anesthetized with a 40-80 mg/kg IM injection of a mixture of 85/15 Ketamine HCl 100 mg/ml and Xylazine HCl 20 mg/ml.

The experiment was carried out as follows:

The rats were shaved and a 6 cm incision was marked on the skin overlaying the linea on the ventral midline. The animals' skin was prepped with iodofor solution and incised. The skin was retracted and undermined slightly to facilitate suturing at the end of the procedure. With the muscle wall exposed, a 5 cm incision in the muscle was made along the linea all through the peritoneal cavity. The right abdominal wall was reflected. A 2 cm x 1 cm of the peritoneum was removed. The medial edge of this defect was located 1 cm lateral from the midline incision and parallel to it. The abdominal wall defect was exposed to air for 10 minutes to monitor any bleeding.

The wounds were sprayed with one of the fibrin glue preparations (EVICEL without any protease inhibitor addition or with tranexamic acid or aprotinin as indicated above). In each rat, 0.5 ml of the each component (1 ml of total sealant) was sprayed onto the created defect inside a 2x3 cm mold. 1 minute after spraying, the mold was removed. The incision and the skin were closed with a running 3-0 Sofsilk (Syneture, Cat No. SS-684). The animals in the different groups were sacrificed 1, 3, 5, 7, 9 and 11 days after the initiation of the experiment using CO₂. A V-shape incision was made exposing the abdominal wall, the remainings of the clots in all the sacrificed animals were extracted and weighed.

Each clot was washed with saline, placed into a test tube containing clot solubilizing solution (a volume in the range of 0.5-10 ml depending on the clots' size; 10 ml on the first day and 0.5-ml on the last day; 7M urea and 0.2M NaOH in a PBS-sodium chloride 0.9% buffer mixed at a ratio of 1:2). The test tube was left to stand at room temperature for at least 4 hours until the clot has been completely dissolved, as judged by visual inspection.

Clottable protein concentration in the remaining clot of each sample following clot solubilization was quantitatively determined by the following procedure. 100 µl solubilized clot solution was diluted in 900 µl of Double distilled water (1:10) and read at 280 nm. The measurements were carried out in 1 ml cuvettes. The clot protein was determined after reduction of light scattering at 320 nm and interpolation from a known internal standard. The actual volume of the clot solubilizing solution used to dissolve the clot remains was taken into account for calculation of the protein amount.

### Example 1- Preparation of cells embedded in fibrin scaffolds.

Plated cells prepared as described above under "Preparation of cell culture" were trypsinzed, washed, pelleted by centrifugation and 40,000 cells were re-suspended in 0.5 ml PBS to wash any trypsin remnants. Suspended cells were pelleted again in a centrifuge at 1000g for 4 minutes at room temperature (about 22°C), and the supernatant was discarded. The pelleted cells were then seeded inside a fibrin scaffold prepared as follows: The above pellet was mixed with 10 µl of thrombin solution (a solution as in the thrombin component of EVICEL^{™} fibrin sealant, Omrix Biopharmaceuticals Ltd., but diluted to a concentration of 20 IU/ml in calcium based buffer: 110 mM mannitol, 150 mM sodium chloride, 20 mM sodium acetate, 6 mg/ml Human Serum Albumin, 40 mM calcium chloride). The mixture of thrombin and cells was mixed with a 10 µl drop of a solution comprising different concentrations of fibrinogen (the stock of fibrinogen solution used is the BAC component of EVICEL^{™} fibrin sealant, Omrix Biopharmaceuticals Ltd., and is diluted to a concentration of 1, 10, 30, 50 or 70 mg/ml in glycine based buffer: 120 mM sodium chloride, 10 mM trisodium citrate, 120 mM glycine, 95 mM arginine hydrochloride, 1 mM calcium chloride) inside a 24-well plate culture dish. The mixed 20 µl drop was allowed to polymerize for 2 minutes in a sterile hood. The formed fibrin scaffold contained fibrinogen at a final concentration of 0.5, 5, 15, 25 or 35 mg/ml and thrombin at a final concentration of 10 IU/ml. Next, 1 ml of medium according to the cell type as detailed above) were added into the well and the culture dish was incubated at 37°C and 5% CO₂. The cells were cultured inside the fibrin scaffold for 1-9 days. During this period several parameters were evaluated (Examples 2-6). Unless indicated otherwise, the medium was replaced every 3 days. Unless indicated otherwise, the volumes and concentration written in this example were used.

### Example 2- The effect of fibrinogen concentration on the ability to support formation of a three-dimensional tissue scaffold.

In the following experiments, three types of cells were used to test the effect of fibrinogen concentration in supporting a three-dimensional tissue scaffold: Human Umbilical Vein Endothelial Cells (HUVEC), Human Mesenchymal Stem Cells (MSC), and human Umbilical Tissue Derived Cells (hUTC) as specified above. These cells originate from different cell lineages. MSC are multipotent stem cells that can differentiate into adipocytes, chondrocytes, endothelial cells and osteoblasts; HUVEC are endothelial stem cells from newborn origin; and hUTC are human umbilical tissue-derived cells that secrete a variety of growth factors including angiogenesis modulating growth factors. These cells are postpartum-derived. The cells produce at least one of CD10, CD13, CD44, CD73, CD90, PDGFr-alpha, PD-L2 and HLA-A,B,C, markers and lack of production of at least one of CD31, CD34, CD45, CD80, CD86, CD117, CD141, CD 178, B7-H2, HLA-G and HLA-DR, DP, DQ, markers as detected by flow cytometry. Each cell type was embedded in fibrin as described in Example 1 and cultured for a period of 7 days. At this point, a phase contrast image of the cells was taken in a magnification of X10 and the formation of a three-dimensional scaffold with embedded cells was assessed. The experiment was carried out in duplicates.

Fig. 1, 2 and 3 show a representative phase contrast image of the different cell types (HUVEC, hUTC, MSC, respectively) in the different fibrinogen concentrations used to prepare the fibrin scaffold: 1 (A), 10 (B); 30 (C); 50 (D); or 70 (E) mg/ml (the final fibrinogen concentration following mixing of the two components was half i.e. 0.5, 5, 15, 25 or 35 mg/ml).

The results show that when using HUVEC (Fig. 1) a three-dimensional tissue structure with embedded cells is present at the lowest final fibrinogen concentration tested (0.5 mg/ml). In contrast, when using hUTC and MSC (Fig. 2 and 3) a three-dimensional tissue structure is only observed at relatively high fibrinogen final concentrations of 25 and 35 mg/ml. Using hUTC and MSC and a final fibrinogen concentrations of equal or lower than15 mg/ml did not allow formation of three-dimensional tissue structures and resulted in a monolayer of cells at the bottom of the well.

These results show that MSC and hUTC require specific concentration of fibrinogen in order to sustain formation of a three-dimensional tissue scaffold. While HUVEC formed a three-dimensional tissue scaffold using a low fibrinogen concentration such as 0.5 mg/ml, MSC and hUTC need a fibrinogen concentration of higher than 15 mg/ml.

### Example 3- The effect of a three-dimensional fibrin scaffold on the morphology of hUTC embedded and cultured within the scaffold.

The present experiment was aimed to assess the effect of culturing hUTC in a three-dimensional fibrin scaffold on the morphology adopted by the cultured cells.

For this purpose, a three-dimensional scaffold of fibrin including hUTC was formed (as in Example 1) using equal volumes of 50 mg/ml fibrinogen and 20 IU/ml thrombin (resulting in 25 mg/ml and 10 IU/ml final concentrations, respectively) and the morphology adopted by the cultured cells was monitored.

Processes and vessel-like structure formation are morphological changes used as markers for angiogenesis (see Material and methods section for details on morphological change measurements).

Fig. 4 shows a representative microscope phase contrast image (obtained at X20 magnification) of hUTC cultured within a fibrin scaffold formed from 50 mg/ml fibrinogen and 20 IU/ml thrombin (25 mg/ml and 10 IU/ml final, respectively). The figure was taken after 2 or 6 days of culture within fibrin (Fig. 4B, C respectively) or without fibrin (Fig. 4A).

It can be observed in Fig.4 that hUTC cultured within a three-dimensional scaffold of fibrin formed from 50 mg/ml fibrinogen acquired cell processes (striped arrow) and vessel-like structures. The cells cultured in the absence of fibrin scaffolds did not show cell processes (continuous arrow). This result show that the combination of hUTC in 3D scaffolds of fibrin, formed with 25 mg/ml fibrinogen (final), induced an angiogenic effect on the embedded cells. This result indicate that hUTC embedded in three-dimensional scaffold of fibrin formed from 50 mg/ml fibrinogen can be advantageously used to increase vascularization e.g. in ischemic regions.

### Example 4- The effect of fibrinogen concentration in the fibrin scaffold on processes formation and length in hUTC and MSC embedded and cultured within the scaffold.

The previous experiment showed that hUTC cultured within a fibrin scaffold formed from a final fibrinogen concentration of 25 mg/ml acquired cell processes and vessel-like structures (see Fig. 4B-C) (as opposed to hUTC cultured in the absence of fibrin- Fig. 4A). The following experiment was carried out in order to test the effect of fibrinogen concentration used to form the fibrin scaffold on the ability of HUVEC, hUTC, and MSC to form processes. For this purpose, HUVEC, hUTC, or MSC were embedded in fibrin scaffolds prepared with different concentrations of fibrinogen and cultured as described in Example 1. After the cells were cultured in the different scaffolds, the process number and the length of the processes were evaluated as indicated in the above Materials and methods section.

Fig. 5 shows the number of cell process formed per field when HUVEC (A), hUTC (C), and MSC (B) were cultured in fibrin scaffold formed from increasing fibrinogen concentrations (1-70 mg/ml; 0.5-35 mg/ml final).

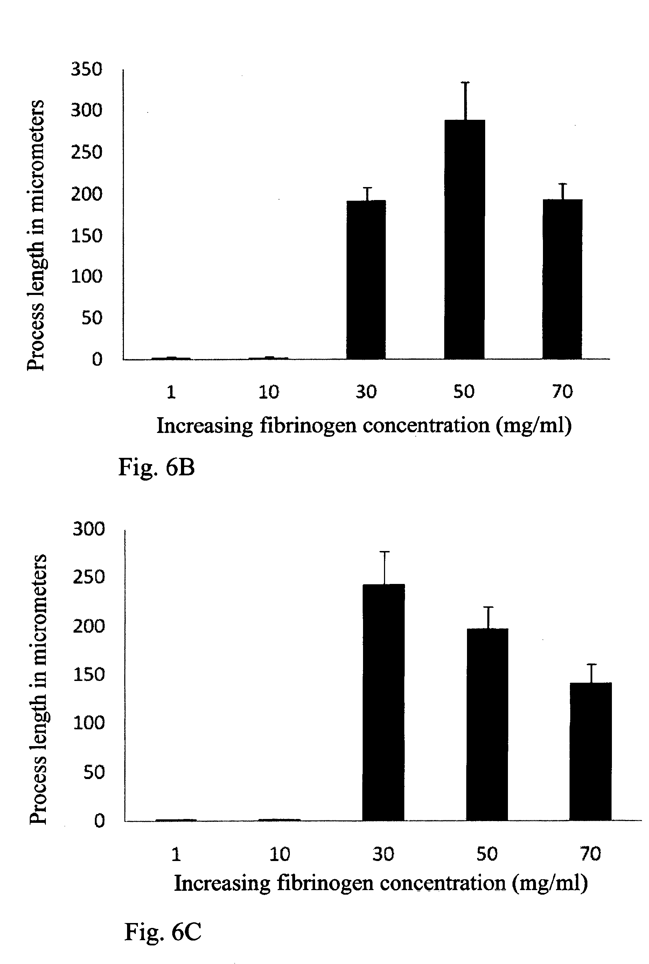

Fig. 6 shows the average length of acquired processes in HUVEC (A), hUTC (C), and MSC (B) cultured in fibrin scaffold formed from increasing fibrinogen concentrations (1-70 mg/ml; 0.5-35 mg/ml final).

It was found that in HUVEC (Fig. 5A) processes can be frequently found in all fibrinogen concentration used to form the scaffold (1-70 mg/ml). The optimal concentration range of fibrinogen for these cells was found to be 10-30 mg/ml fibrin based scaffold (final 5-15 mg/ml). In contrast, in the case of hUTC and MSC (Fig. 5C and B, respectively) it was found that processes are rarely present in scaffolds formed with such low fibrinogen concentration. When using these cells, the frequency of processes increased in scaffolds formed with high fibrinogen concentrations of ≥30 mg/ml (final equal or above 15 mg/ml). With regards to the processes length, it was observed that in HUVEC (Fig. 6A) a similar length is obtained at all fibrinogen concentration used (1-70 mg/ml) whereas in the case of hUTC and MSC (Fig. 6C, B) a fibrinogen concentration of equal to or higher than 30 mg/ml resulted in significantly longer processes as compared to using lower fibrinogen concentration of < 30 mg/ml (< 15 mg/ml final).

These results show that in order to obtain a vessel-like structure which can be beneficial in blood vessel formation, e.g. in ischemic regions, MSC and hUTC require a scaffold formed with a final fibrinogen concentration of at least 15 mg/ml whereas in the case of HUVEC lower fibrinogen concentrations are optimal.

### Example 5- The effect of the various cell types embedded within the fibrin scaffold on the level of fibrin degradation.

The previous examples showed that hUTC and MSC require a fibrin scaffold formed with a relatively high fibrinogen concentration, as compared to HUVEC, in order to obtain three-dimensional vessel-like structures.

The following experiment was designed to explore the possibility of differential fibrin degradation rate attributed to each cell type cultured within the fibrin scaffold.

D-dimer is a fibrin degradation product formed when fibrin polymer is broken down by the enzyme plasmin. Fibrin scaffold degradation was assessed by measuring the D-dimer level in the medium of various cells cultured within fibrin scaffolds.

For this purpose, HUVEC, MSC or hUTC were grown in a fibrin scaffold as described in Example 1. A sample (20 µl) was taken from the medium 1 and 7 days following seeding of the cells and the D-dimer level was measured using D-dimer ELISA Kit (Imuclone, USA, Cat No. 602) according to the manufacturer's protocol. The medium was not replaced during the entire experiment period. Fibrin scaffolds without cells and fibrin scaffolds with cells on day 1 served as control groups.

Fig. 7A-7B show the D-dimer concentration after 1 (A) and 7 (B) days in the medium of HUVEC, MSC and hUTC grown in fibrin scaffold formed with increasing fibrinogen concentrations (and in fibrin scaffold without cells as control).

It was found that in hUTC and MSC a significantly higher (3-5 fold) D-dimer concentration is present in the medium 7 days following culturing as compared to the D-dimer level in the medium of cultured HUVEC, indicating that hUTC and MSC have a high capability level of degrading fibrin. This high capability level of fibrin degradation by hUTC and MSC is probably a result of a higher plasmin enzymatic activity level i.e. a higher level of plasmin is secreted by these cells.

These results indicate that in order to obtain a long lasting three-dimensional fibrin scaffold when seeding hUTC and MSC within a fibrin scaffold, a fibrin polymer with high fibrinogen content should be used especially when the fibrin sealant used does not contains anti-fibrinolytic agents. It should be noted that the use of anti-fibrinolytic agents such as Aprotinin or Tranexamic acid has been shown to be cytotoxic (Furtmüller et al. "Tranexamic acid, a widely used antifibrinolytic agent, causes convulsions by a gamma-aminobutyric acid(A) receptor antagonistic effect". J Pharmacol Exp Ther. 2002;301:168-173; Roger et al. "Evaluating the differences between fibrin sealants: recommendations from an international advisory panel of hospital pharmacists". The European Journal of Hospital Pharmacy Science Volume 12,2006, Issue 1, P. 3-9).

### Example 6- The ability of fibrin scaffolds to support cell expansion.

Cells seeded in a fibrin scaffold may have a limited capacity to proliferate in a certain cell seeding concentration. Thus, the present experiment was carried out to examine what is the optimal cell concentration for initial seeding which can still be supported in the fibrin scaffold.

Increasing numbers of cells of the various cell types: 1,000-10,000 cells per µl fibrin scaffold in total of 30 µl fibrin scaffold/well were seeded in a 6 well-plate within a fibrin scaffold or in the absence of fibrin (and in this case the cells were grown in monolayer located on the bottom of the well plate). Cells were embedded in the fibrin scaffolds in the same manner as exemplified above (Example 1), except that the scaffolds were generated from 15 µl fibrinogen component in a concentration of 50 mg/ml and 15 µl thrombin component in the concentration of 20 IU/ml (a final concentration of 25 mg/ml and 10 IU/ml, respectively). The well plates were incubated at 37°C in a humidified atmosphere of 5% CO₂ for 1.5 days. Afterwards, the medium was discarded and the cells were counted as described in the "Material and methods section". Cells in the monolayer culture were counted by the Hoechst nuclear stain method as described in the Material and methods section.

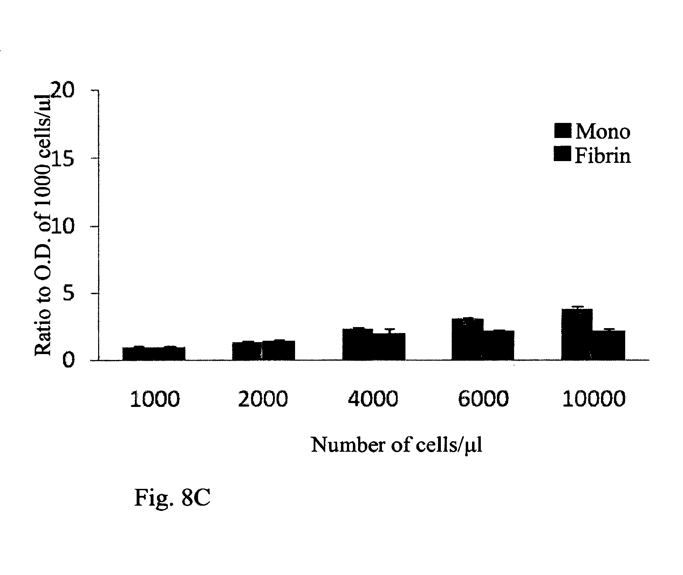

The results are shown in Fig. 8A-C. As expected, it was observed that when HUVEC (Fig. 8A), MSC (Fig. 8B) and hUTC (Fig. 8C) are cultured as a monolayer an increase in the number of seeded cells resulted in a proportional increase in the amount of cells present following 1.5 days of culturing. In HUVEC (Fig. 8A) an increase in the number of seeded cells within the fibrin scaffold resulted in a proportional increase in the number of cells following 1.5 days incubation. In contrast, in MSC and hUTC the numbers of cells did not increase significantly at the same proportion after the same incubation period in some of the higher initial high number of cell seed. For example, MSC seeded beyond 6,000 cell/µl or hUTC seeded beyond 4,000 cells/µl did not result in a proportional increase in the number of cells following the incubation period of 1.5 days as compared to HUVEC. These findings suggest that in MSC and hUTC the optimal initial seeding concentration range is more stringent as compared to that of HUVEC and it is crucial for the ability of the MSC and hUTC to grow and proliferate within the fibrin scaffold.

### Example 7- Retention of MSC, hUTC, or HUVEC cells embedded in fibrin scaffolds in a limb muscle animal model.

The purpose of this experiment was to examine the ability of fibrin scaffold to retain cells at the injected site. The mouse limb muscle cell injection model was used as an *in vivo* model system as described in the "Material and methods" section.

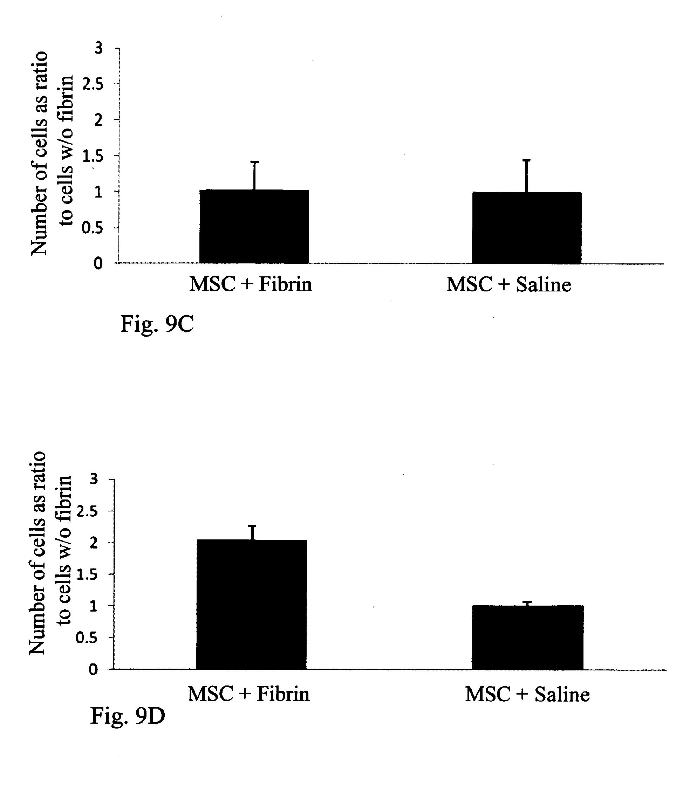

The results are summarized in Fig. 9. The Y axis shows the number of cells per a given microscope field (1000x1000 microns) counted in the limb injected with fibrin + cells or number of cells counted in the limb injected with cells + saline divided by the number of cells counted in the limb injected with cells + saline. This number represents the ratio of (injected cells + fibrin) to (injected cells in saline).

Figs. 9A-9D show the number of cells present in the injected muscle after 1 day (A, C) and 4 days (B, D) of injection of fibrin + cells {formed with either 17.5 mg/ml fibrinogen final (A) (B)] or 25 mg/ml fibrinogen final (C) (D)} or injection of saline + cells. The counted number of cells in either of the fibrin + cells injected limb or the saline + cells injected limb was divided by the number of cells counted in the saline + cells injected limb on the same day, to obtain a ratio of cells in either limb.

In the figure only MSC are depicted, yet similar results were observed with all other cells tested (hUTC and HUVEC) (data not shown). The results show that injection of cells with a thrombin component and a fibrinogen component in a concentration of 50 mg/ml (25 mg/ml final) resulted in a significant increase in the retention of cells in the injected region 4 days following implantation of the cells as compared to cells injected with saline. In contrast, when a fibrinogen component in a lower concentration of 35 mg/ml (17.5 mg/ml final) was used no significant increase in retention was observed as compared to cells injected with saline (Fig. 9).

These results show that a fibrin scaffold formed with a fibrinogen component at a concentration of higher than 35 mg/ml (17.5 mg/ml final) need to be used to form a cell scaffold that anchors the cells to the injected region e.g. a muscle tissue for a therapeutically period of time and thus can be advantageously used in tissue engineering.

### Example 8- The effect of added protease inhibitor on fibrin clot longevity in an in vivo setting.

The purpose of this experiment was to test the effect of added protease inhibitors (tranexamic acid or aprotinin) on the longevity of the fibrin clot in an *in vivo* setting. The rat abdominal wall defect model was used as elaborated above. For this purpose, three different fibrin sealant compositions (EVICEL without any protease inhibitor addition or with tranexamic acid or aprotinin as indicated above) were applied onto a defect created in a rat abdominal muscle wall (see details in the "Material and Methods" section). The animals in the different groups were sacrificed 1, 3, 5, 7, 9 and 11 days after the initiation of the experiment and longevity of the clot was assessed by measuring the clot's weight and the clottable protein amount as specified above.

The mean clot weight and the mean clottable protein amount of the various fibrin sealant compositions at the various time points are presented in Fig. 10 and 11, respectively.

The results obtained indicate that the clot weight (Fig. 10) and the clottable protein amount (Fig. 11) declines with time in all tested groups. No significant differences were found between the groups regarding the clot's weight and the clottable protein amount. These results demonstrate that the addition of protease inhibitor into a fibrin scaffold in an *in vivo* setting does alter clot longevity. Therefore, addition of protease inhibitors to fibrin scaffolds alone cannot prolong the residence of the embedded cells in the implantation site.

## Claims

1. A fibrin based scaffold capable of supporting a population of cells embedded in the scaffold comprising an initial concentration of cells of at least 1 x 10⁶ cells/ml scaffold mesenchymal stem cells (MSC) and/or umbilical tissue derived cells (UTC), wherein the scaffold is formed with a fibrinogen component comprising fibrinogen having a final concentration of higher than 17.5 mg/ml scaffold, the scaffold comprising embedded UTC; MSC; or a combination thereof at an initial concentration range of 1 x 10⁶ to 6 x 10⁶ cells/ml scaffold, wherein the fibrin scaffold is substantially free of added protease inhibitors, and wherein the scaffold comprising embedded MSC and/or UTC is for use in increasing vascularisation.

2. The scaffold according to claim 1, wherein the fibrinogen component is blood derived, and preferably the scaffold is substantially free of plasmin and/or plasminogen.

3. UTC and/or MSC for use in a method of increasing vascularization at a confined region of a subject in need wherein the method comprises administering the cells to the region as part of a scaffold according to claim 1 or claim 2.

4. The UTC and/or MSC for use in a method according to claim 3, wherein the region is ischemic, damaged or comprises a transplanted tissue or organ.

5. The UTC and/or MSC for use in a method according to claim 3 or claim 4, wherein the region is a muscle tissue.

6. UTC and/or MSC for use in a method for treating a wound wherein the method comprises administering or forming proximal to the wound a scaffold according to claim 1 or claim 2 which comprises embedded UTC and/or MSC.

7. The UTC and/or MSC for use in a method according to claim 6, wherein the scaffold is capable of supporting the cells at the wound or proximal to the wound for a therapeutically effective period of time, preferably wherein the time is at least 4 days.

8. A method for preparing a scaffold for use in increasing vascularisation, said method comprising the steps of: providing a fibrinogen component; providing a proteolytic enzyme component which is capable of forming fibrin when it reacts with fibrinogen; providing a population of cells comprising MSC and/or UTC; and mixing the fibrinogen, the proteolytic enzyme, and the cells, wherein the formed scaffold comprises 1 x 10⁶ to 6 x 10⁶ cells/ml scaffold MSC and/or UTC, wherein the fibrinogen component used to form the scaffold is in a final concentration of higher than 17.5 mg/ml scaffold and wherein the components are substantially free of added protease inhibitors.

9. The method according to claim 8, wherein the fibrinogen component is blood derived and is substantially free of plasmin and/or plasminogen.

10. A scaffold according to claim 1 or claim 2 which comprises embedded UTC and/or MSC or a scaffold formed according to claim 8 or claim 9 for use in increasing vascularization at a confined region in a subject and/or for use in treating a wound.

11. A kit for use in increasing vascularisation comprising:
(i) at least two separated containers comprising components required to form a fibrin based scaffold, the at least one separated container comprises a fibrinogen component having a final fibrinogen concentration of higher than 17.5 mg/ml scaffold formed, and the at least second separated container comprises a proteolytic enzyme component which is capable of forming fibrin when it reacts with fibrinogen; and
(ii) MSC and/or UTC at a concentration range of 1 x 10⁶ to 6 x 10⁶ cells/ml scaffold formed, wherein the components are substantially free of added protease inhibitors.

12. The kit according to claim 11, wherein the MSC and/or UTC are in a separated container.

13. The kit according to claim 11 or claim 12, wherein the fibrinogen component is blood derived and is substantially free of plasmin and/or plasminogen.

## Patentansprüche

1. Gerüst auf Fibrinbasis, das fähig ist, eine Population von Zellen zu tragen, die in dem Gerüst eingebettet ist und eine Anfangskonzentration von Zellen von wenigstens 1 x 10⁶ Zellen/ml Gerüst an mesenchymalen Stammzellen (MSC) und/oder aus Umbilikalgewebe abgeleiteten Zellen (UTC) umfasst, wobei das Gerüst mit einer Fibrinogen-Komponente gebildet ist, die Fibriniogen mit einer Endkonzentration von mehr als 17,5 mg/ml Gerüst umfasst, wobei das Gerüst eingebettete UTC; MSC; oder eine Kombination davon mit einer Anfangskonzentration im Bereich von 1 x 10⁶ bis 6 x 10⁶ Zellen/ml Gerüst umfasst, wobei das Fibringerüst im Wesentlichen frei von zugegebenen Proteasehemmern ist und wobei das Gerüst, das eingebettete MSC und/oder UTC umfasst, für die Verwendung zum Erhöhen der Vaskularisation ist.

2. Gerüst gemäß Anspruch 1, wobei die Fibrinogenkomponente aus Blut abgeleitet ist und vorzugsweise das Gerüst im Wesentlichen frei von Plasmin und/oder Plasminogen ist.

3. UTC und/oder MSC für die Verwendung bei einem Verfahren zum Erhöhen der Vaskularisation in einem beschränkten Bereich eines Subjekts mit Bedarf daran, wobei das Verfahren Verabreichen der Zellen an den Bereich als Teil eines Gerüsts gemäß Anspruch 1 oder Anspruch 2 ist.

4. UTC und/oder MSC für die Verwendung bei einem Verfahren gemäß Anspruch 3, wobei der Bereich ischämisch ist, geschädigt ist oder ein transplantiertes Gewebe oder Organ umfasst.

5. UTC und/oder MSC für die Verwendung bei einem Verfahren gemäß Anspruch 3 oder Anspruch 4, wobei der Bereich ein Muskelgewebe ist.

6. UTC und/oder MSC für die Verwendung bei einem Verfahren zum Behandeln einer Wunde, wobei das Verfahren das Verabreichen eines Gerüsts gemäß Anspruch 1 oder Anspruch 2, das eingebettete UTC und/oder MSC umfasst, an die Wunde oder Bilden davon nahe der Wunde umfasst.

7. UTC und/oder MSC für die Verwendung bei einem Verfahren gemäß Anspruch 6, wobei das Gerüst fähig ist, die Zellen an der Wunde oder nahe der Wunde über einen therapeutisch wirksamen Zeitraum zu tragen, vorzugsweise wobei der Zeitraum wenigstens 4 Tage beträgt.

8. Verfahren zum Herstellen eines Gerüsts für die Verwendung zum Erhöhen der Vaskularisation, wobei das Verfahren die Schritte umfasst: Bereitstellen einer Fibrinogenkomponente; Bereitstellen einer proteolytischen Enzymkomponente, die fähig ist, Fibrin zu bilden, wenn sie sich mit Fibrinogen umsetzt; Bereitstellen einer Population von Zellen, die MSC und/oder UTC umfasst; und Mischen des Fibrinogens, des proteolytischen Enzyms und der Zellen, wobei das gebildete Gerüst 1 x 10⁶ bis 6 x 10⁶ Zellen/ml Gerüst an MSC und/oder UTC umfasst, wobei die zum Bilden des Gerüsts verwendete Fibrinogenkomponente in einer Endkonzentration von mehr als 17,5 mg/ml Gerüst vorliegt und wobei die Komponenten im Wesentlichen frei von zugegebenen Proteasehemmern sind.

9. Verfahren gemäß Anspruch 8, wobei die Fibrinogenkomponente aus Blut abgeleitet ist und im Wesentlichen frei von Plasmin und/oder Plasminogen ist.

10. Gerüst gemäß Anspruch 1 oder Anspruch 2, umfassend eingebettete UTC und/oder MSC, oder gemäß Anspruch 8 oder Anspruch 9 gebildetes Gerüst für die Verwendung zum Erhöhen der Vaskularisation in einem beschränkten Bereich in einem Subjekt und/oder für die Verwendung zum Behandeln einer Wunde.

11. Kit für die Verwendung zum Erhöhen der Vaskularisation, umfassend:
(i) wenigstens zwei gesonderte Behälter, umfassend Komponenten, die zum Bilden eines Gerüsts auf Fibrinbasis erforderlich sind, wobei der wenigstens eine gesonderte Behälter eine Fibrinogenkomponente mit einer Fibrinogen-Endkonzentration von über 17,5 mg/ml gebildetes Gerüst umfasst und der wenigstens zweite gesonderte Behälter eine proteolytische Enzymkomponente umfasst, die fähig ist, Fibrin zu bilden, wenn sie sich mit Fibrinogen umsetzt; und
(ii) MSC und/oder UTC in einem Konzentrationsbereich von 1 x 10⁶ bis 6 x 10⁶ Zellen/ml gebildetes Gerüst, wobei die Komponenten im Wesentlichen frei von zugegebenen Proteasehemmern sind.

12. Kit gemäß Anspruch 11, wobei sich die MSC und/oder UTC in einem gesonderten Behälter befinden.

13. Kit gemäß Anspruch 11 oder Anspruch 12, wobei die Fibrinogenkomponente aus Blut abgeleitet ist und im Wesentlichen frei von Plasmin und/oder Plasminogen ist.

## Revendications

1. Échafaudage à base de fibrine capable de soutenir une population de cellules incorporées dans l'échafaudage comprenant une concentration initiale de cellules d'au moins 1 x 10⁶ cellules/ml d'échafaudage de cellules souches mésenchymateuses (MSC) et/ou de cellules dérivées de tissu ombilical (UTC), l'échafaudage étant formé avec un composant de fibrinogène comprenant du fibrinogène ayant une concentration finale supérieure à 17,5 mg/ml d'échafaudage, l'échafaudage comprenant des UTC incorporées ; des MSC incorporées ; ou une combinaison de celles-ci dans une plage de concentration initiale de 1 x 10⁶ à 6 x 10⁶ cellules/ml d'échafaudage, l'échafaudage de fibrine étant sensiblement exempt d'inhibiteurs de protéase ajoutés, et l'échafaudage comprenant des MSC et/ou UTC incorporées étant pour utilisation dans l'augmentation de la vascularisation.

2. Échafaudage selon la revendication 1, dans lequel le composant de fibrinogène est dérivé de sang, et de préférence, l'échafaudage est sensiblement exempt de plasmine et/ou de plasminogène.

3. UTC et/ou MSC pour utilisation dans un procédé d'augmentation de la vascularisation dans une région confinée d'un sujet nécessitant cela, le procédé comprenant l'administration des cellules à la région sous forme de partie d'un échafaudage selon la revendication 1 ou la revendication 2.

4. UTC et/ou MSC pour utilisation dans un procédé selon la revendication 3, la région étant ischémique, endommagée ou comprenant un tissu ou un organe transplanté.

5. UTC et/ou MSC pour utilisation dans un procédé selon la revendication 3 ou la revendication 4, la région étant un tissu musculaire.

6. UTC et/ou MSC pour utilisation dans un procédé pour traiter une plaie, le procédé comprenant l'administration ou la formation à proximité de la plaie d'un échafaudage selon la revendication 1 ou la revendication 2 qui comprend des UTC et/ou MSC incorporées.

7. UTC et/ou MSC pour utilisation dans un procédé selon la revendication 6, l'échafaudage étant capable de soutenir les cellules au niveau de la plaie ou à proximité de la plaie pendant une durée thérapeutiquement efficace, de préférence la durée étant d'au moins 4 jours.

8. Procédé de préparation d'un échafaudage pour utilisation dans l'augmentation de la vascularisation, ledit procédé comprenant les étapes de : fourniture d'un composant de fibrinogène ; fourniture d'un composant d'enzyme protéolytique qui est capable de former de la fibrine lorsqu'il réagit avec le fibrinogène ; la fourniture d'une population de cellules comprenant des MSC et/ou UTC ; et mélange du fibrinogène, de l'enzyme protéolytique, et des cellules, l'échafaudage formé comprenant de 1 x 10⁶ à 6 x 10⁶ cellules/ml d'échafaudage de MSC et/ou UTC, le composant de fibrinogène utilisé pour former l'échafaudage étant à une concentration finale supérieure à 17,5 mg/ml d'échafaudage et les composants étant sensiblement exempts d'inhibiteurs de protéase ajoutés.

9. Procédé selon la revendication 8, dans lequel le composant de fibrinogène est dérivé de sang et est sensiblement exempt de plasmine et/ou de plasminogène.

10. Échafaudage selon la revendication 1 ou la revendication 2 qui comprend des UTC et/ou MSC incorporées ou échafaudage formé selon la revendication 8 ou la revendication 9 pour utilisation dans l'augmentation de la vascularisation dans une région confinée chez un sujet et/ou pour utilisation dans le traitement d'une plaie.

11. Kit pour utilisation dans l'augmentation de la vascularisation comprenant :
(i) au moins deux récipients séparé comprenant des composants requis pour former un échafaudage à base de fibrine, l'au moins un récipient séparé comprend un composant de fibrinogène ayant une concentration finale de fibrinogène supérieure à 17,5 mg/ml d'échafaudage formé, et l'au moins un deuxième récipient séparé comprend un composant d'enzyme protéolytique qui est capable de former de la fibrine lorsqu'il réagit avec le fibrinogène ; et
(ii) des MSC et/ou UTC dans une plage de concentration de 1 x 10⁶ à 6 x 10⁶ cellules/ml d'échafaudage formé, dans lequel les composants sont sensiblement exempts d'inhibiteurs de protéase ajoutés.

12. Kit selon la revendication 11, dans lequel les MSC et/ou UTC sont dans un récipient séparé.

13. Kit selon la revendication 11 ou la revendication 12, dans lequel le composant de fibrinogène est dérivé de sang et est sensiblement exempt de plasmine et/ou de plasminogène.
